# EUROPEAN PATENT SPECIFICATION

(11) **EP 4 000 688 B1**
(45) Date of publication and mention of the grant of the patent: **05.11.2025**
(21) Application number: 20208625.2
(22) Date of filing: 19.11.2020
(51) Int. Cl.: A61P 5/26, A61P 21/06, C07J 1/00

(54) **PROCESS FOR THE PREPARATION OF TRENBOLONE ACETATE HAVING A DEFINITE PARTICLE SIZE DISTRIBUTION**
VERFAHREN ZUR HERSTELLUNG VON TENBOLONACETAT MIT BESTIMMTER PARTIKELGRÖSSENVERTEILUNG
PROCÉDÉ POUR LA PRÉPARATION D'ACÉTATE TRENBOLONE AYANT UNE DISTRIBUTION GRANULOMÉTRIQUE DÉFINIE

(43) Date of publication of application: 25.05.2022
(73) Proprietor: F.I.S.- Fabbrica Italiana Sintetici S.p.A., 36075 Montecchio Maggiore (VI) (IT)
(72) Inventor: Rencurosi, Anna, 36075 Montecchio Maggiore (VI) (IT); Brescello, Roberto, 36075 Montecchio Maggiore (VI) (IT)
(74) Representative: Leganza, Alessandro

(56) References cited:
- WO-A2-2007/106768
- CN-A- 102 399 253
- CN-A- 102 924 553
- CN-A- 108 017 682
- DD-A1- 134 769
- GB-A- 1 035 683
- US-A1- 2004 258 589
- ZHANG HUYUE ET AL: "Synthesis of Trenbolone acetate in high yield", ACTA ACADEMIAE MEDICINAE SHANGHAI, SHANGHAI, CN, vol. 29, no. 3, 1 January 2002 (2002-01-01), pages 211 - 212, XP008084590, ISSN: 0257-8131

## Description

### Technical Field

The present invention refers to an improved process for the preparation of Trenbolone Acetate. Moreover, it is also related to the PSD of Trenbolone Acetate.

### Background Art

Trenbolone Acetate is a small molecule, sold under brand names such as Finajet and Finaplix among others, is an androgen and anabolic steroid (AAS) medication which is used in veterinary medicine, specifically to increase the profitability of livestock by promoting muscle growth in cattle.

The drug is a synthetic androgen and anabolic steroid and hence is an agonist of the androgen receptor (AR), the biological target of androgens like testosterone and dihydrotestosterone (DHT). It has strong anabolic effects and highly androgenic effects, as well as potent progestogenic effects, and weak glucocorticoid effects. Trenbolone acetate is an androgen ester and a long-lasting prodrug of trenbolone in the body.

Trenbolone acetate was discovered in 1963 and was introduced for veterinary use in the early 1970s.

Trenbolone acetate, or trenbolone 17β-acetate, is a synthetic estrane steroid and a derivative of nandrolone (19-nortestosterone). It is the C17β acetate ester of trenbolone, which itself is δ9,11-19-nortestosterone (δ9,11-19-NT) or estra-4,9,11-trien-17β-ol-3-one. Other trenbolone esters include trenbolone enanthate, trenbolone hexahydrobenzylcarbonate, and trenbolone undecanoate.

Tronbolone acetate has the following chemical formula (I): 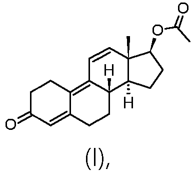 and chemical name is 17beta-acetoxy-estra-4,9,11-triene-3-one.

The described synthetic route of Trenbolone Acetate is long, and some reaction yield is extremely low, so that the final product crystallization is very difficult.

Zhang et al. (in Fudan Xuebao, Yixueban 2002, 29(3), 211-212) discloses a preparation method of trenbolone acetate. The method comprises the following steps: taking estra-4,9-dien-3,17-dione (VI) as a starting raw material, carrying out protection on carbonyl group in the 3-position with methanol and p-toluenesulfonic acid as catalyst, afterwards the carbonyl group in the 17-position is reduct to hydroxy group with sodium boro hydride. The obtaining product (IV) is deprotect in acid condition to restor the carbonyl group in 3-position. The obtaining (17β)-17-Hydroxyestra-5,9-dien-3-one (III) is converted tu the Trenbolone (II) by oxidation with 2,3-Dichloro-5,6-dicyano-1,4-benzoquinone (DDQ). In the last step is carry out the esterification reaction on the hydroxyl group in 17-position of Trenbolone by acetic anhydride to obtain Trenbolone acetate (I), wherein the specific route is as follows:

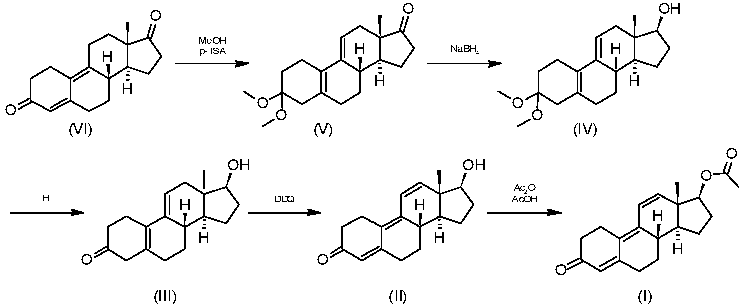

The described route has many and long steps and multiple byproducts, solid products cannot be obtained, the quality is low, the yield is low, high-toxicity solvent benzene is needed, and industrial production is not facilitated.

According to the route disclosed by the Chinese patent CN102399253, (17β)-17-Hydroxyestra-4,9-dien-3-one (VII) is used as a raw material. Diacetyl enol ester (VIII) was obtaind by acetiletation with acetyl chloride and acetic anhydride. Afterwards an hydrolysis of acetyl group in position 3 and an oxidative dehydrogenation are carried out to obtain the Trenbolone acetate, and the specific route is as follows:

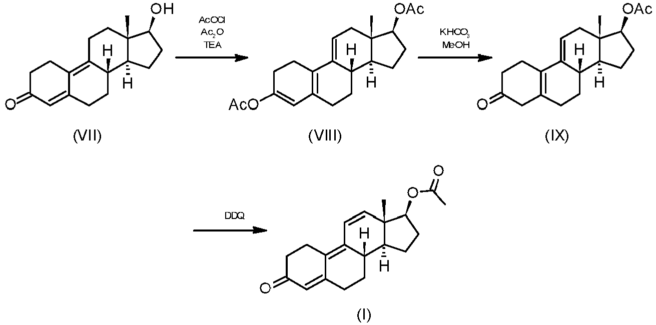

The route is firstly subjected to 17-position hydroxyl esterification, the next enol-esterification of the carbonyl group in 3 position is difficult and have a low conversion. The successive selective hydrolysis of the enol ester in 3 position is difficult, excessive hydrolysis of the acetyl group in 17-position is easy to occur. The purification of obtaining intermediate (IX) is difficult, and the scale up of process is difficult.

The route of the Chinese patent CN102924553 describe a optimized process of Zhang et al. wherein the acidic catalyst in the protection of carbonyl group in 3-position is changed from p-toluenesulfonic acid to acetyl chloride/methanol. This route uses a large amount of acidic reagent i.e. acetyl chloride, which implies large corrosion, the requirement on the production equipment is high, for which corrosion resistant equipment is required. Consequently, the waste amount is large, and the total yield is not significatilvely improved.

The route disclosed by the Chinese patent CN108017682 start from a conpound have a 4,9 open rings. Through a reduction with potassium borohydride, and a subsequent acid-catalyzed condensation, the (17β)-17-Hydroxyestra-5,9-dien-3-one (III) is obtaind. The next oxidative dehydrogenation with DDQ and the esterification, the Trenbolone acetate is obtained, and the specific route is as follows:

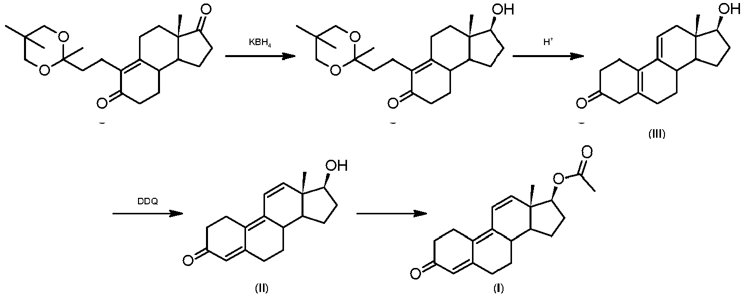

The potassium-boron hydrogen reduction is unselective and difficut to control; moreover the acid-catalyzed ring closing condensation have a low yield. Finaly, the intermediates and the product require a purification because have a low quality.

The route disclosed by the Chinese patent CN110437294 is very similar to the route discoser by Zhang et al. (in Fudan Xuebao, Yixueban 2002, 29(3), 211-212). the differences between the two ways lie in the protector group used to protect the carbonyl group in position 3, from a methanol acetal to a ethylenedioxy acetal.

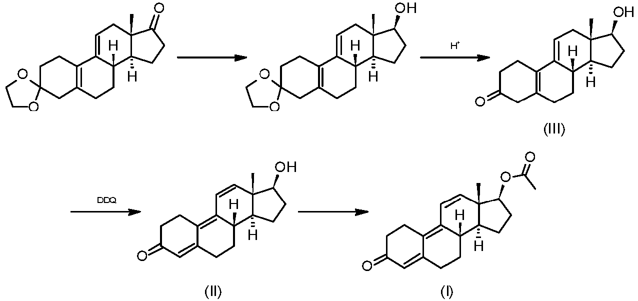

Furthermore, CN-A-108 017 682, CN-A-102 924 553, CN-A-102 399 253, Fudan Xuebao, Yixueban Vol 29(3), pp 211-212 (2002), GB-A-1,035,683 and DD134769 all disclose the crystallisation of Trenbolone Acetate from various solvents, including isopropyl ether (iPr₂O), ethyl acetate (EtOAc) and mixtures containing iPr₂O or EtOAc, but *not* isopropanol.

All of these prior art methods for the preparation of Trenbolone Acetate do not describe a preparation method for the crystallization of trenbolone Acetate heaving a define PSD of the obtaining product.

Furthermore, the prior art methods have the drawbacks related to the poor reproducibility of the process and of the solid form thus obtained.

Thus, there is a need for a process for preparing the intermediate Trenbolone and the final product Trenbolone Acetate in high yield and quality.

### Summary of invention

The problem addressed by the present invention is therefore that of providing a better process for crystallization of Trenbolone Acetate, which allows to get round to the drawbacks above reported with reference to the known prior art.

Moreover, the present invention is therefore that of providing a process for the preparation of Trenbolone Acetate having a median value (D50) of the particle size distribution as defined below and in claim 1 , by means of a reproducible process.

This problem is solved by a process for the crystallization of Trenbolone Acetate as outlined in the annexed claims, whose definitions are integral part of the present description.

Particularly, the present invention provides a process for the preparation of Trenbolone Acetate of formula (I):

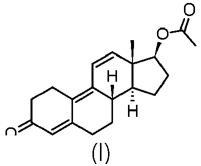

having a median value (D50) of the particle size distribution comprised in the range as defined below and in claim 1, by means of crystallization of Trenbolone Acetate by means of addition of a seed heaving a define median value (D50) of the particle size distribution.

As a further aspect, the process provides an improved solid form of Trenbolone Acetate, said form being Trenbolone Acetate having a defined PSD this feature allows the product to be arranged in a more compact way and therefore have a higher solid density, from which it allows to have more compound per volume unit; i.e. with Trenbolone Acetate of formula (I) having define PSD is possible to have more active ingredient for the same volume of pharmaceutical composition. However, this solid form and pharmaceutical compositions thereof, do not form part of the present invention *per se.*

Further features and advantages of the process according to the invention will result from the description hereafter reported of examples of realization of the invention, provided as an indication of the invention.

### Drawings

Fig. 1 shows the powder x-ray diffraction pattern of compound of formula (I), obtained by the process of the present invention.
Fig. 2 shows the DSC curve of compound of formula (I), obtained by the process of the present invention.

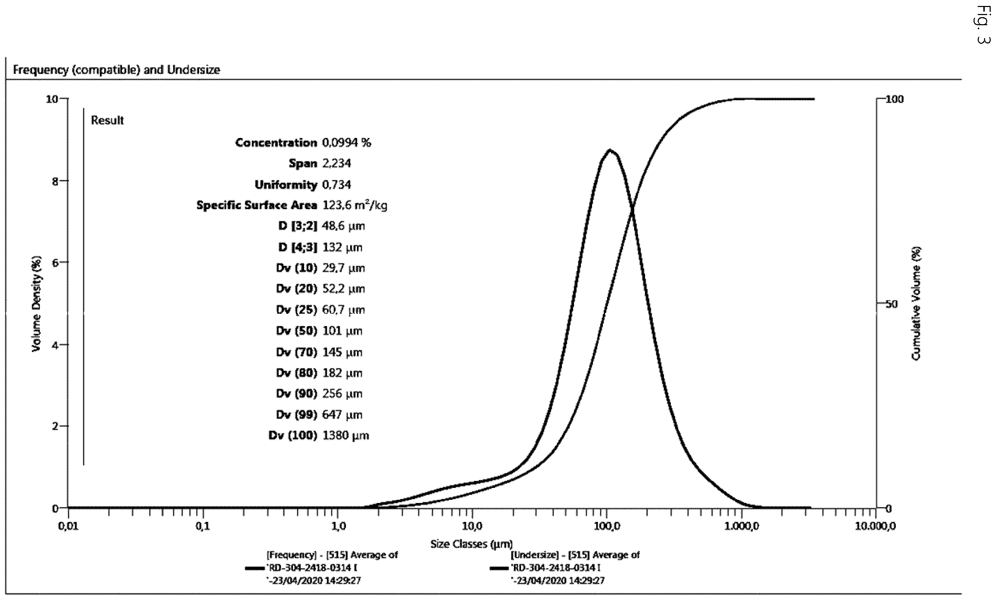

Fig. 3 shows the tipical PSD distrivution of compound of formula (I), obtained by the process of the present invention.

### Description of embodiments

A mean particle size means the measure relates to mean diameter of the as determined by laser diffraction.

The present invention is related to a process for the preparation of Trenbolone Acetate of formula (I): 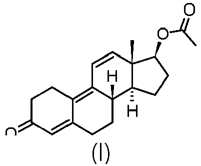 having a median value (D50) of the particle size distribution comprised in the range from 50 µm to 80 µm, or from 60 µm to 90 µm, or from 90 µm to 250 µm, by means of addition of a seed having a median value (D50) of the particle size distribution comprised respectively in the range from 8 µm to 15 µm, or from 15 µm to 20 µm, or from 20 µm to 90 µm, to a solution of Trenbolone Acetate in a solvent, wherein said process comprises
the following steps:
a) providing a solution of Trenbolone Acetate in a solvent or solvents mixture;
b) seeding said solution with a Trenbolone Acetate seed having a median value (D50) of the particle size distribution comprised in the range from 8 µm to 15 µm, or from 15 µm to 20 µm, or from 20 µm to 90 µm;
c) cooling until the obtainment of a suspension;
d) filtering the obtained suspension to obtain Trenbolone Acetate of formula (I) having a median value (D50) of the particle size distribution comprised in the range respectively from 50 µm to 80 µm, or from 60 µm to 90 µm, or from 90 µm to 250 µm, wherein the solution of the step (a) is Trenbolone Acetate in isopropanol.

According to the process of the present invention, the seed of the step b) has a median value (D50) of the particle size distribution comprised in the range from from 8 µm to 15 µm, or from 15 µm to 20 µm, or from 20 µm to 90 µm.

According to a preferred embodiment of the process of the present invention, the seed of the step b) has a median value (D50) of the particle size distribution comprised in the range from 8 µm to 40 µm.

The seed of Trenbolone Acetate of formula (I) is an amount from 0.50% w/w to 1.5% w/w referred to the entire amount of Trenbolone Acetate (I).

According to a preferred embodiment of the process of the present invention, the seeding of the step b) is carried out at a temperature comprised from 20°C to 30°C.

According to a more preferred embodiment of the process of the present invention, the seeding of the step b) is carried out at a temperature comprised from 20°C to 25°C.

According to a preferred embodiment of the process of the present invention,the temperature during the cooling of the step c) decreases from a temperature comprised in the range from 20°C to 40°C to a temperature comprised in the range from of -20°C to 0°C.

According to a more preferred embodiment of the process of the present invention,the temperature during the cooling of the step c) decreases from a temperature comprised in the range from 20°C to 30°C to a temperature comprised in the range from of -20°C to -10°C.

According to a more preferred embodiment of the process of the present invention,the temperature during the cooling of the step c) decreases from a temperature comprised in the range from 20°C to 26°C to a temperature comprised in the range from of -18°C to -10°C.

According to a preferred embodiment of the process of the present invention, the temperature during the crystallization of Trenbolone Acetate of formula (I) decreases from a temperature in the range from 20°C to 40°C to the temperature of -20°C in 8 hours.

In the step d) Trenbolone Acetate of formula (I) is isolated, for example, by filtration or centrifugation.

The molar yield of the process according to the present invention starting from Trenbolone Acetate of formula (I) is comprised between 85% and 95%.

The molar yield of the process according to the more preferred embodiment of the present invention starting from Trenbolone Acetate of formula (I) is comprised between 90% and 95%.

According to a preferred embodiment, the process of the invention provide Trenbolone Acetate of formula (I) having high chemical purity, i.e. more than 99.50% A/A%.

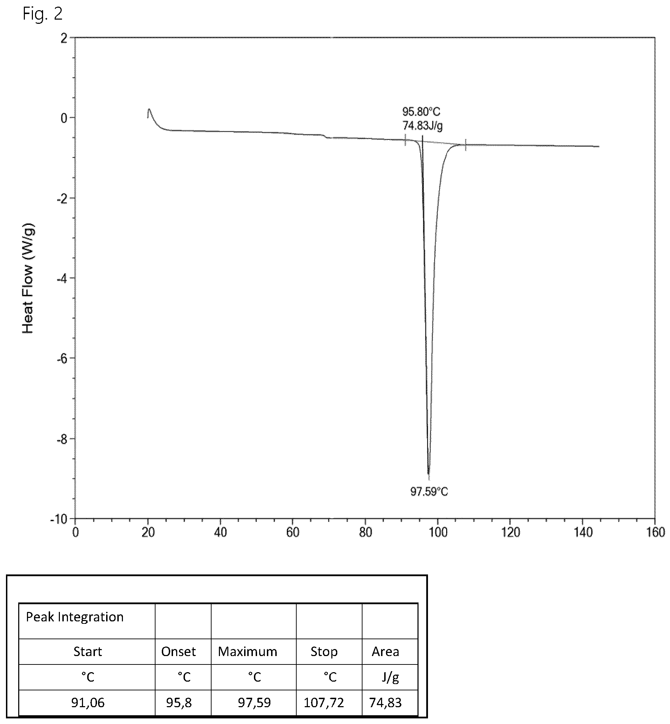

The solid form of Trenbolone Acetate having a median value (D50) of the particle size distribution comprised in the range from 50 µm to 80 µm, or from 60 µm to 90 µm, or from 90 µm to 250 µm, by means of addition of a seed having a median value (D50) of the particle size distribution comprised respectively in the range from 8 µm to 15 µm, or from 15 µm to 20 µm, or from 20 µm to 90 µm, has a melting point of 95.8°C as measured by DSC (onset) (See Fig. 2).

Specifically, the value of the DSC onset and DSC peak is recorded as measured by DSC.

The DSC onset corresponds to the melting point recorded by DSC analysis, whose method is better described in the experimental part.

Moreover, Trenbolone Acetate (I) of the invention shows a peak at 97.9°C as measured by DSC.

In particular, Trembolone Acetate having a median value (D50) of the particle size distribution comprised in the range from 50 µm to 80 µm, or from 60 µm to 90 µm, or from 90 µm to 250 µm, by means of addition of a seed having a median value (D50) of the particle size distribution comprised respectively in the range from 8 µm to 15 µm, or from 15 µm to 20 µm, or from 20 µm to 90 µm, has a characteristic X-ray powder diffraction pattern with characteristic peaks expressed in 2-Theta values (2θ) at: 6.5, 13.2, 20.9 each peak ± 0.2 .

More in particular, Trenbolone Acetate having a median value (D50) of the particle size distribution comprised in the range from 50 µm to 80 µm, or from 60 µm to 90 µm, or from 90 µm to 250 µm, by means of addition of a seed having a median value (D50) of the particle size distribution comprised respectively in the range from 8 µm to 15 µm, or from 15 µm to 20 µm, or from 20 µm to 90 µm, has a characteristic X-ray powder diffraction pattern with characteristic peaks expressed in 2-Theta values (2θ) at: 6.5, 13.2, 16.1, 19.5, 20.9, 22.3 each peak ± 0.2.

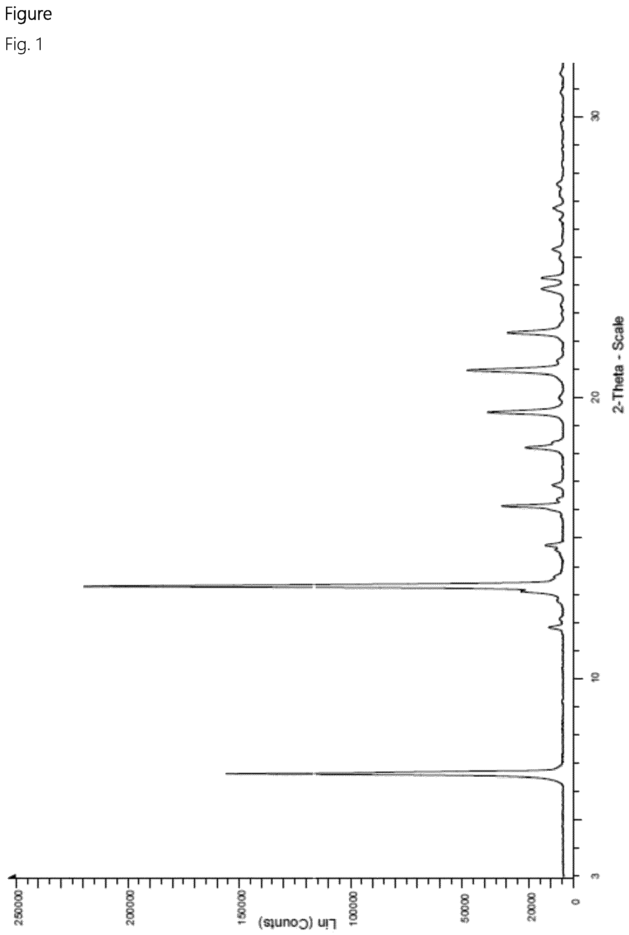

Again more particularly, Trenbolone Acetate having a median value (D50) of the particle size distribution comprised in the range from 50 µm to 80 µm, or from 60 µm to 90 µm, or from 90 µm to 250 µm, by means of addition of a seed having a median value (D50) of the particle size distribution comprised respectively in the range from 8 µm to 15 µm, or from 15 µm to 20 µm, or from 20 µm to 90 µm, has a characteristic X-ray powder diffraction pattern with characteristic peaks expressed in 2-Theta values (2θ) at: 6.5, 13.2, 16.1, 18.2, 19.5, 20.9, 22.3, 23.8 and 24.2, each peak ± 0.2 (See Fig. 1).

In another embodiment the present invention encompasses process for preparing of Trenbolone Acetate (I), comprising the previous step of preparation of the compound of formula (I), by acetylation of Trenbolone of formula (II):

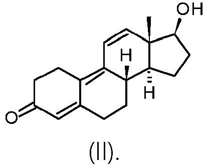

According to a preferred embodiment of the process of the present invention, the ancetylation step of Trenbolone of formula (II) and the crystallization of the Trenbolone Acetate (I) are carried out one-pot.

Specifically, the process of the invention can be carried out one-pot, i.e. starting from step of acetylation of Trenbolone of formula (II) and continuing to step of crystallization, thus producing a solid Trenbolone Acetate of formula (I), keeping the compound of Trenbolone Acetate (I) obtained in step of acetylation in a solution, i.e. without isolating it in a solid form.

The molar yield of the process according to the present invention starting from Trenbolone of formula (II) is comprised between 75% and 90%.

The molar yield of the process according to more preferred embodiment of the present invention starting from Trenbolone of formula (II) is comprised between 80% and 87%.

According to a preferred embodiment of the process of the present invention, Trenbolone Acetate of formula (I): 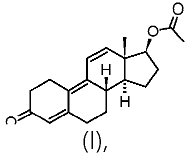 has a median value (D50) of the particle size distribution comprised in the range from 50 µm to 130 µm.

In another embodiment the present invention encompasses process for preparing of Trenbolone Acetate (I), which is has a median value (D50) of the particle size distribution comprised in the range from 50 µm to 130 µm.

According to a preferred embodiment of the process of the present invention, Trenbolone Acetate (I), which is has a median value (D50) of the particle size distribution comprised in the range from 70 µm to 100 µm.

According to a preferred embodiment of the process of the present invention, Trenbolone Acetate of formula (I) having a D10 of the particle size distribution less than 40 µm.

According to a preferred embodiment of the process of the present invention, Trenbolone Acetate (I), which is has a median value (D10) of the particle size distribution comprised in the range from 15 µm to 40 µm.

According to a preferred embodiment of the process of the present invention, Trenbolone Acetate of formual (I) having a D90 of the particle size distribution less than 300 µm.

According to a more preferred embodiment of the process of the present invention, Trenbolone Acetate of formual (I) having a D90 of the particle size distribution less than 200 µm.

The solid form of Trenbolone Acetate produced by the process of the present invention has a median value (D50) of the particle size distribution comprised in the range from 50 µm to 130 µm, has a melting point of 95.8°C as measured by DSC (onset) (See Fig. 1). However, this solid form does not form part of the present invention *per se.*

In particular, Trembolone Acetate produced by the process of the present invention has a median value (D50) of the particle size distribution comprised in the range from 50 µm to 130 µm, has a characteristic X-ray powder diffraction pattern (i.e. XPRD) with characteristic peaks expressed in 2-Theta values (2θ) at: 6.5, 13.2, 20.9 each peak ± 0.2 . However, this solid form does not form part of the present invention *per se.*

More in particular, Trenbolone Acetate produced by the process of the pefsent invention has a median value (D50) of the particle size distribution comprised in the range from 50 to 130 µm, has a characteristic XPRD pattern with characteristic peaks expressed in 2-Theta values (2θ) at: 6.5, 13.2, 16.1, 19.5, 20.9, 22.3 each peak ± 0.2. However, this solid form does not form part of the present invention *per se.*

Again more particularly, Trenbolone Acetate produced by the process of the present invention has a median value (D50) of the particle size distribution comprised in the range from 50 µm to 130 µm, has a characteristic X-ray powder diffraction pattern with characteristic peaks expressed in 2-Theta values (2θ) at: 6.5, 13.2, 16.1, 18.2, 19.5, 20.9, 22.3, 23.8 and 24.2, each peak ± 0.2 (See Fig. 1). However, this solid form does not form part of the present invention per se.

Disclosed herein, but not forming part of the present invention, is thus Trenbolone Acetate of formula (I): 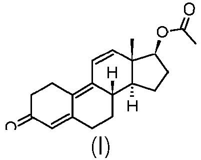 having irregular hexagon plates crystal habit wherein said Trenbolone Acetate (I) is in the form of only plates crystals.

In another embodiment the present invention encompasses process for preparing of Trenbolone Acetate of formula (I), which has an irregular hexagon plates crystal habit.

Indeed the process of the present invention allows the production of Trenbolone Acetate having irregular hexagon plates crystal habit in the form of only plates crystals. It means that said solid form does not contain crystals having different morphology, but contains only plates crystals.

Trenbolone Acetate having irregular hexagon plates crystal habit in the form of only plates crystals thus does not contain any soft agglomerate or any other crystal having different morphology.

Moreover, Trenbolone Acetate having irregular hexagon plates crystal habit is an homogeneous solid.

Trenbolone Acetate having irregular hexagon plates crystal habit and which is in the form of only plate crystals can be suitably employed for the preparation of pharmaceutical compositions.

Disclosed herein, but not forming part of the present invention, are pharmaceutical compositions comprising Trenbolone Acetate of formula (I): 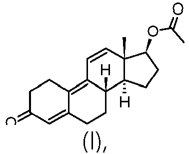 has a median value (D50) of the particle size distribution comprised in the range from 50 to 130 µm and one or more pharmaceutical acceptable excipients.

Disclosed herein, but not forming part of the present invention, are pharmaceutical compositions comprising Trenbolone Acetate of formula (I) having a D10 of the particle size distribution less than 40 µm and one or more pharmaceutical acceptable excipients.

Disclosed herein, but not forming part of the present invention, are pharmaceutical compositions comprising Trenbolone Acetate of formula (I) having a D90 of the particle size distribution less than 300 µm and one or more pharmaceutical acceptable excipients..

Examples of suitable pharmaceutical compositions comprising Trenbolone Acetate according to the present invention are those disclosed in WO 2001043748A2, WO 9930685 A1, WO 9947073 A1, WO 2000025743 A2, WO 2001043749 A2, WO 2019217203 A1 or WO 2020061550 A1 with the difference that Trenbolone Acetate having has a median value (D50) of the particle size distribution comprised in the range from 50 µm to 130 µm.

Pharmaceutical compositions have different dosage forms, which may include, for example, capsules, tablets, powders, suspensions or any other suitable dosage form. In such said dosage forms, the Trenbolone Acetate (I) having hexagon plates crystal habit may be combined with one or more pharmaceutically acceptable excipients, carriers or diluents, such as, for example mannitol, silicic derivatives or sugar.

Trenbolone Acetate of formula (I) having a median value (D50) of the particle size distribution comprised in the range from 50 µm to 130 µm, and/or a D10 of the particle size distribution less than 40 µm, and /or a D90 of the particle size distribution less than 300 µm, as above described, can be used for the preparation of a pharmaceutical composition. However, such pharmaceutical compositions do not form part of the present invention.

Trenbolone Acetate of formula (I) having a median value (D50) of the particle size distribution comprised in the range from 50 µm to 80 µm, or from 60 µm to 90 µm, or from 90 µm to 250 µm, by means of addition of a seed having a median value (D50) of the particle size distribution comprised respectively in the range from 8 µm to 15 µm, or from 15 µm to 20 µm, or from 20 µm to 90 µm, as above described, can be used for the preparation of a pharmaceutical composition.

Trenbolone Acetate of formula (I) having a median value (D50) of the particle size distribution comprised in the range from 50 µm to 130 µm, and/or a D10 of the particle size distribution less than 40 µm, and /or a D90 of the particle size distribution less than 300 µm, as above described, or the beforehand above mentioned pharmaceutical composition can be used as medicament or for use in medicine or use in veterinary.

Trenbolone Acetate of formula (I) having a median value (D50) of the particle size distribution comprised in the range from 50 µm to 80 µm, or from 60 µm to 90 µm, or from 90 µm to 250 µm, by means of addition of a seed having a median value (D50) of the particle size distribution comprised respectively in the range from 8 µm to 15 µm, or from 15 µm to 20 µm, or from 20 µm to 90 µm, as above described, or the beforehand above mentioned pharmaceutical composition can be used as medicament or for use in medicine or use in veterinary.

Trenbolone Acetate of formula (I) having a median value (D50) of the particle size distribution comprised in the range from 50 µm to 80 µm, or from 60 µm to 90 µm, or from 90 µm to 250 µm, by means of addition of a seed having a median value (D50) of the particle size distribution comprised respectively in the range from 8 µm to 15 µm, or from 15 µm to 20 µm, or from 20 µm to 90 µm, as above described, or the beforehand above mentioned pharmaceutical composition can be used to promote androgen and/or increase muscle mass in cattle.

Disclosed herein, but not forming part of the present invention, is Trenbolone Acetate of formula (I) having a median value (D50) of the particle size distribution comprised in the range from 50 to 130 µm, and/or a D10 of the particle size distribution less than 40 µm, and /or a D90 of the particle size distribution less than 300 µm. In such a case, especially, where the crystal does not facilitates the manufacturing process of the pharmaceutical product, the small particle size distribution provides a very positive effect, thus balancing the two effects, and providing a product which is the more stable solid form of Trenbolone Acetate.

Disclosed herein, but not forming part of the present invention, is Trenbolone Acetate having a median value (D50) of the particle size distribution comprised in the range from 50 µm to 80 µm, or from 60 µm to 90 µm, or from 90 µm to 250 µm, by means of addition of a seed having a median value (D50) of the particle size distribution comprised respectively in the range from 8 µm to 15 µm, or from 15 µm to 20 µm, or from 20 µm to 90 µm. In such a case, especially, where the crystal does not facilitates the manufacturing process of the pharmaceutical product, the small particle size distribution provides a very positive effect, thus balancing the two effects, and providing a product which is the more stable solid form of Trenbolone Acetate.

Disclosed herein, but not forming part of the present invention, is Trenbolone Acetate of formula (I) having a median value (D50) of the particle size distribution comprised in the range from 50 µm to 130 µm, and/or a D10 of the particle size distribution less than 40 µm, and /or a D90 of the particle size distribution less than 300 µm. Such a product is particularly homogenous and therefore appears to be the easiest product to be used in the manufacturing process for preparing the pharmaceutical product. Furthermore it allows a better control of the amount of the active substance dosed during process for preparing the pharmaceutical product.

Disclosed herein, but not forming part of the present invention, is Trenbolone Acetate, having a median value (D50) of the particle size distribution comprised in the range from 50 µm to 80 µm, or from 60 µm to 90 µm, or from 90 µm to 250 µm, by means of addition of a seed having a median value (D50) of the particle size distribution comprised respectively in the range from 8 µm to 15 µm, or from 15 µm to 20 µm, or from 20 µm to 90 µm. Such a product is particularly homogenous and therefore appears to be the easiest product to be used in the manufacturing process for preparing the pharmaceutical product. Furthermore it allows a better control of the amount of the active substance dosed during process for preparing the pharmaceutical product.

Disclosed herein, but not forming part of the present invention, is a pharmaceutical composition comprising Trenbolone Acetate of formula (I) having a median value (D50) of the particle size distribution comprised in the range from 50 µm to 130 µm, and/or a D10 of the particle size distribution less than 40 µm, and /or a D90 of the particle size distribution less than 300 µm, and one or more pharmaceutical acceptable excipients can be prepared.

Disclosed herein, but not forming part of the present invention, is a pharmaceutical composition comprising Trenbolone Acetate of formula (I) having a median value (D50) of the particle size distribution comprised in the range from 50 µm to 80 µm, or from 60 µm to 90 µm, or from 90 µm to 250 µm, by means of addition of a seed having a median value (D50) of the particle size distribution comprised respectively in the range from 8 µm to 15 µm, or from 15 µm to 20 µm, or from 20 µm to 90 µm, and one or more pharmaceutical acceptable excipients can be prepared.

Said pharmaceutical compositions, not forming part of the present invention, which contain Trenbolone Acetate of formula (I) having a median value (D50) of the particle size distribution comprised in the range from 50 µm to 130 µm, and/or a D10 of the particle size distribution less than 40 µm, and /or a D90 of the particle size distribution less than 300 µm, can have the same administration route and dosage forms as beforehand described related to pharmaceutical composition comprising Trenbolone Acetate of the prior art.

Said pharmaceutical compositions, not forming part of the present invention, which contain Trenbolone Acetate of formula (I) having a median value (D50) of the particle size distribution comprised in the range from 50 µm to 80 µm, or from 60 µm to 90 µm, or from 90 µm to 250 µm, by means of addition of a seed having a median value (D50) of the particle size distribution comprised respectively in the range from 8 µm to 15 µm, or from 15 µm to 20 µm, or from 20 µm to 90 µm, can have the same administration route and dosage forms as beforehand described related to pharmaceutical composition comprising Trenbolone Acetate of the prior art.

Disclosed herein, but not forming part of the present invention, are pharmaceutical compositions comprising Trenbolone Acetate of formula (I): 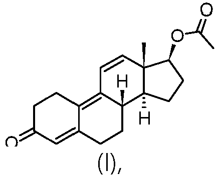 prepared according to the process of the invention and one or more pharmaceutical acceptable excipients.

According to a preferred embodiment, the process for the preparation of Trenbolone Acetate of formula (I) provides said compound of formula (I) having has a median value (D50) of the particle size distribution comprised in the range from 50 µm to 130 µm.

Disclosed herein, but only forming part of the present invention in as far as it also incorporates the process of the invention as defined above and in the claims, is the preparation of said pharmaceutical compositions, the process the following steps:
a) providing the said Trenbolone Acetate of the invention;
b) mixing the Trenbolone Acetate of step a) with a one or more pharmaceutical acceptable excipients;
c) packaging the obtained mixture of step b).

Trenbolone Acetate of formula (I) having a median value (D50) of the particle size distribution comprised in the range from 50 µm to 130 µm, and/or a D10 of the particle size distribution less than 40 µm, and /or a D90 of the particle size distribution less than 300 µm, can be more easily formulated into pharmaceutical compositions of medicaments.

Trenbolone Acetate having a median value (D50) of the particle size distribution comprised in the range from 50 to 80 µm, or from 60 to 90 µm, or from 90 to 250 µm, by means of addition of a seed having a median value (D50) of the particle size distribution comprised respectively in the range from 8 to 15 µm, or from 15 to 20 µm, or from 20 to 90 µm, can be more easily formulated into pharmaceutical compositions of medicaments.

The process of the present invention is carried out by means of a crystallization of Trenbolone Acetate of formula (I). The crystallization, in the present case, is a process of formation of solid crystals from a solution wherein Trenbolone Acetate of formula (I) has been previously prepared and/or solubilized. In particular the term crystallization of the process of the present invention means either crystallization or recrystallization.

The recrystallization is a technique used for purifying chemical compounds and/or for obtaining a different solid forms (e.g. polymorphs), in particular a solid compound is solubilized in an appropriate solvent, and then the compound re-become a solid, typically a solid crystal, by means of heating and then cooling treatment.

The process of the present invention can be thus, for example, carried out starting from Trenbolone Acetate and recrystallizing it by heating/cooling treatment or, alternatively, can be carried out by obtaining Trenbolone Acetate in solution and then crystallizing the product by cooling.

Moreover, said improved process for the preparation of Trenbolone Acetate of formula (I) having a median value (D50) of the particle size distribution comprised in the range from 50 µm to 130 µm, and/or a D10 of the particle size distribution less than 40 µm, and /or a D90 of the particle size distribution less than 300 µm, allows the preparation of Trenbolone Acetate having high chemical purity, i.e. more than 99.90% by HPLC A/A%.

Moreover, said improved process for the preparation of Trenbolone Acetate having a median value (D50) of the particle size distribution comprised in the range from 50 to 80 µm, or from 60 to 90 µm, or from 90 to 250 µm, by means of addition of a seed having a median value (D50) of the particle size distribution comprised respectively in the range from 8 to 15 µm, or from 15 to 20 µm, or from 20 to 90 µm allows the preparation of Trenbolone Acetate having high chemical purity, i.e. more than 99.90% by HPLC A/A%.

It has been indeed surprisingly found that the crystallization of Trenbolone Acetate (I), having a median value (D50) of the particle size distribution comprised in the range from 50 to 80 µm, or from 60 to 90 µm, or from 90 to 250 µm, by means of addition of a seed having a median value (D50) of the particle size distribution comprised respectively in the range from 8 to 15 µm, or from 15 to 20 µm, or from 20 to 90 µm, is a well reproducible and solid process for obtaining Trenbolone Acetate a median value (D50) of the particle size distribution comprised in the range from 50 µm to 130 µm.

Moreover, Trenbolone Acetate of formula (I) having a median value (D50) of the particle size distribution comprised in the range from 50 µm to 130 µm, this feature allows the product to be arranged in a more compact way and therefore have a higher solid density, from which it allows to have more compound per volume unit; i.e. with Trenbolone Acetate of formula (I) having a median value (D50) of the particle size distribution comprised in the range from 50 µm to 130 µm, is possible to have more active ingredient for the same volume of pharmaceutical composition.

Another surprising advantage of the process of the invention is given, contrary to what is observed in the processes of the prior art, by the ease of controlling the precipitation and also by the non-aggregation of the crystallized product (i.e. no lumps are observed in the product)

It has been indeed surprisingly found that the crystallization of Trenbolone Acetate of formula (I), according to the process of the invention, is well reproducible and solid process for obtaining Trenbolone Acetate a median value (D50) of the particle size distribution comprised in the range from 50 µm to 130 µm.

All the features and preferred embodiments of the process of the present invention given above can be combined in each possible combination to carry out the claimed process, provided that these result in a process possessing all of the essential features of the process of the invention as defined above and in claim 1.

Moreover, the person skilled in the art of organic chemistry can appreciate as the process of the invention allows an improvement of the solid density, this feature allows the product to be arranged in a more compact, from which it allows to have more compound per volume unit; i.e. with Trenbolone Acetate of formula (I) having a median value (D50) of the particle size distribution comprised in the range from 50 µm to 130 µm, is possible to have more active ingredient for the same volume of pharmaceutical composition.

### EXPERIMENTAL SECTION

The starting material Trenbolone and Trenbolone Acetate can be prepared according to well-known prior art methods, or for example, as described in the CN 108017682, CN 102399253, CN 102924553 or in CN 110437294 or can be purchased on the market.

Suspension means a solid material suspended in a solvent or solution, i.e. mixture of a solid with a solvent, which is liquid. The solvent also contains other compound or a solid.

Room temperature (RT) means a temperature that is comprised in a range from 20 to 25°C, it is defined as comfortable temperature range indoors.

Molar equivalent means that the molar amount of a substance reacts with a molar amount of another substance in a given chemical reaction.

The term "volume" means volume of solvent per unit of product, thus, for example, 1 volume is 1 Liter per 1 Kilo, or 1 mL per 1 gram, or 1 microliter per 1 milligram. Thus, 10 volumes means for example 10 liters per 1 Kilogram of substance.

Example 1: Preparation of Trenbolone, starting from 9(10)-Dehydronandrolone (DHN).

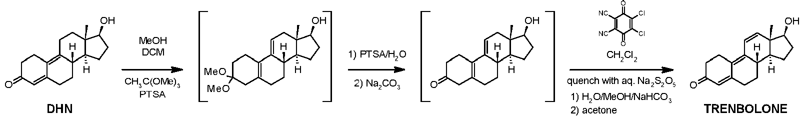

To a solution of 100 g DHN in 250 mL DCM, 50 mL MeOH and 1.4 g of PTSA is added dropwise over the course of at least 2h to a stirred solution of 100 mL DCM, 350 mL MeOH and 61 mL trimethyl orthoacetate. At the end of the addition, the addition funnel is rinsed with 33 mL DCM and the rinse transferred into the reaction mixture. After IPC analysis showing reaction completion, to the mixture was added 500 mL of water and left stirring for 0.5-2 h. The aqueous phase is discarded and to the organic phase is added a mixture of 400 mL water and 5 g PTSA. After IPC analysis showing reaction completion, the biphasic mixture is separated, and the organic phase is neutralized by addition under stirring at r.t. of a solution of water (100 mL) and NaHCO₃ (0.2 g), which brings the pH at 7-8. The phases are separated, and to the organic phase are added 200 mL DCM. The obtained solution is concentrated under vacuum to around 200 mL by keeping the internal temperature below 30 °C. This addition / concentration protocol is repeated until the solution is anhydrous. After reaching this limit, the mixture is evaporated one last time until the total volume reaches 300 mL. The mixture is treated with 24 mL of acetic acid and added dropwise to a stirred suspension of 96 g DDQ in 700 mL DCM, maintaining the internal temperature between 0-7 °C. After the end of the addition, the addition vessel is rinsed with 50 mL DCM and the rinse is combined with the reaction mixture, which is left stirring at 0-7 °C until reaching complete conversion. The reaction mixture is quenched by addition at 0-7°C of a solution composed of 38 g of aqueous Na₂S₂O₅ (30% by weight), 73 mL water and 20 mL MeOH. The obtained slurry is warmed to r.t., left stirring for 0.5 h after which it is filtered, and the filter cake is washed with 2x100 mL DCM. The obtained biphasic mixture is separated, and the organic phase washed with a solution of 200 mL water, 20 mL MeOH and 7.7 g of NaHCO₃. After phase separation, the organic layer is washed again with a solution of 200 mL water, 60 mL MeOH and 7.7 g of NaHCO₃. The phases are separated, and the organic layer washed one final time with a solution of 200 mL water, 100mL MeOH and 7.7 g NaHCO₃. The combined, filtered solution is concentrated with stirring to 300 mL under vacuum, keeping the internal temperature below 30 °C. To the resulting solution, 300 mL of acetone are added, and the obtained mixture concentrated under vacuum to 300 mL (total volume) again, keeping the internal temperature below 30 °C. The resulting suspension is cooled to 0 °C and kept at this temperature for 0.5 h with stirring, after which it is filtered, and the filter cake is washed twice with 100 mL acetone. The wet solid is dried at 40 °C max under vacuum to give Trenbolone in 65% yield and >99.0 % A/A purity by HPLC.

Example 2: Preparation of Trenbolone Acetate, starting from Trenbolone, , by acetylation following of crystallization from Isopropanol (IPA). 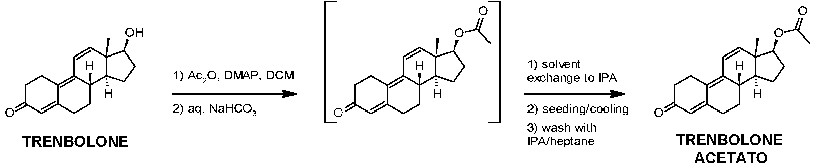 To a stirred mixture of 100 g Trenbolone and 2 g 4-DMAP in 250 mL DCM are added 60 g of acetic anhydride. The anhydride addition funnel is rinsed with 50 mL of DCM and the obtained solution is warmed to 30°C with stirring. At complited reaction, the solution is cooled to 20°C, and added to a suspension of 31 g NaHCO₃ in 300 mL water with stirring. The acetylation reactor is rinsed with additional 50 mL DCM and the rinse is added to the biphasic reaction mixture, which is then warmed again to 30°C with stirring for 1h. Then the mixture is cooled to 20°C, and the aqueous layer is discarded. The organic layer is washed with 100 mL water, and phase are separed. To the obtained organic solution is added isopropanol (300 mL) and the mixture is concentrated under vacuum to 500 mL, with the internal temperature kept at 30°C. To the mixture is added isopropanol (300 mL) again, and the solvent distilled under vacuum to 580 mL (total volume) by keeping the internal temperature at 30°C. This addition/concentration protocol is repeated three times. The solution is seeded with 0.5 g Trenbolone acetate heaving PSD range D10: 4-12 µm; D50: 12-29 µm; D90: <76 µm, at an internal temperature of 20-25°C. The mixture is left at 20-25°C for 30', after which it is cooled slowly to an internal temperature of -20/-10 °C. The obtained suspension is kept at -20/-10°C for 0.5 h and then filtered. The filter cake is washed with 100 mL of cold IPA, and then the resulting wet solid is triturated by resuspension in cold n-heptane (150 mL) for 0.5 h, after which the mixture is filtered and dried at 40°C under vacuum to give Trenbolone acetate in 83% yield and >99.0% A/A by HPLC, with a PSD within D10: 15-40 µm; D50: 67-130 µm; D90: ≤350 µm.

Example 3: Crystallization of Trenbolone Acetate from IPA.

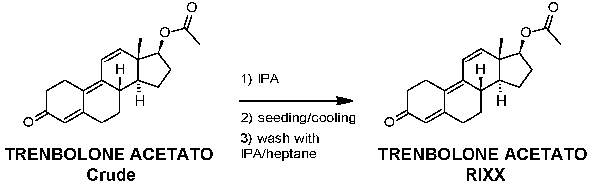

To a suspension of 50 g Trenbolone acetate in 200 mL IPA at 20°C is added methylene chloride (2.5 mL). The mixture is heated to 30°C upon which complete dissolution is observed. The solution is cooled to 20-25°C, and it is seeded with 0.5% w/w of Trenbolone acetate (PSD range D10: 2-5 µm; D50: 10-20 µm; D90: 25-51 µm). The mixture is left at 20-25°C for 30', after which it is cooled slowly to an internal temperature of -15°C. The obtained suspension is kept at -15 °C for 0.5 h and then filtered. The filter cake is washed with 50 mL of cold Ethanol, and then the resulting wet solid is triturated by resuspension in cold n-heptane (75 mL) for 0.5 h, after which the mixture is filtered and dried at 40°C max under vacuum to give Trenbolone acetate in 83% yield and >99.0% A/A by HPLC, with a PSD within D10: 15-40 µm; D50: 67-130 µm; D90: ≤300 µm.

Example 4: The successful experiments that provided Trenbolone Acetate of formula (I) obtained according to the process of the example 3 are collected in table below:

| **Trial** | **PSD seed (µm)** | | | **T seed (°C)** | **Cooling ramp (°C/min)** | **PSD of Compound (I) (µm)** | | |
|---|---|---|---|---|---|---|---|---|
| | **D10** | **D50** | **D90** | | | **D10** | **D50** | **D90** |
| **1** | 32 | 90 | 174 | 22 | 0.1 | 54 | 252 | 633 |
| **2** | 32 | 90 | 174 | 25 | 0.1 | 54 | 169 | 425 |
| **3** | 2.6 | 13.1 | 27 | 23 | 0.1 | 23 | 66 | 145 |
| **4** | 2.6 | 13.1 | 27 | 23 | 0.1 | 20 | 71 | 210 |
| **5** | 2.6 | 13.1 | 27 | 25 | 0.1 | 15.9 | 51.5 | 123 |
| **6** | 2.4 | 12 | 25.3 | 23 | 0.1 | 26.7 | 75.8 | 168 |
| **7** | 2.4 | 12 | 25.3 | 23 | 0.2 | 21.4 | 101 | 258 |
| **8** | 3.9 | 17.7 | 38.5 | 23 | 0.1 | 23 | 72 | 205 |
| **9** | 5 | 20 | 51 | 23 | 0.1 | 19.7 | 60.4 | 166 |
| **10** | 2 | 10 | 21 | 23 | 0.1 | 22 | 65 | 177 |
| **11** | 1.6 | 8.2 | 17 | 23 | 0.1 | 26 | 79 | 220 |
| **12** | 5 | 17 | 40 | 26 | 0.1 | 29 | 90 | 275 |
| **13** | 5 | 17 | 40 | 26 | 0.1 | 31 | 89 | 250 |
| **14** | 5 | 17 | 40 | 26 | 0.1 | 24 | 71 | 194 |
| **15** | 11 | 41 | 125 | 26 | 0.1 | 37 | 141 | 500 |

Example 5: Analytical method to identify and quantify of compound of formula (I), moreover for determining the chemical purity, via HPLC:
- Colum: Hypersil-ODS, 150 × 4.0 mm, 3.0 µm, or equivalent;
- Temp. Column: 25°C;
- Mobile Phase A: Acetonitrile/Methanol/MilliQ water 36.5 : 30 : 33.5 v/v/v;
- Mobile Phase B: Acetonitrile/Methanol 90 : 10 v/v;
- Gradient

| Time (min) | %A | %B |
|---|---|---|
| 0 | 100 | 0 |
| 6 | 100 | 0 |
| 16 | 0 | 100 |
| 26 | 0 | 100 |
| 26.1 | 100 | 0 |
| 30 | 100 | 0 |

- Flow: 1.0 mL/min;
- UV Detector: 229 nm;
- Injection Volume: 5 µL;
- Analysis Time: 26 min;
- Diluent: Acetonitrile/Methanol/MilliQ water/Acetic acid 36.5:30:33.5:0.1 v/v/v/v.

Example 6: Particle size distribution (PSD), in particular, particle size distribution was determined with a Malvern Mastersizer 3000, and the other parameters used are reported in table below:

| Dispersing accessory setting | |
|---|---|
| Sample handing unit | Hydro MV |
| Stirrer speed (rpm) | 2500 |
| Built-in Ultrasound power (%) | OFF |

| Material properties | |
|---|---|
| Particle refractive index | 0 |
| Absorption | 0 |
| Scattering Model | Mie |
| Dispersant name | Water 0.1%wt Tween 80 |
| Dispersant refractive index | 1.33 |
| Analysis sensitivity | Normal |
| Single result mode | No |
| Non spherical particle type | Yes |

| Measurement | |
|---|---|
| Sample measurement time (red and blue) | 20 s (12000 snaps) |
| Background measurement time (red and blue) | 20s (12000 snaps) |
| Background stability time out | 120 s |
| Use background check | Yes |
| Background check limits | [1,200], [20,20] |
| Number of measurements | 4 |
| Delay between measurement | 20 s |
| Result unit | volume |
| Obscuration limit | 12.8 -17,3% |

Particle size distribution of Trenbolone Acetate obtained according to the process of the invention shows a gaussian distribution with the D50 value was from 50 to 130 µm.

## Claims

1. Process for the preparation of Trenbolone Acetate of formula (I): 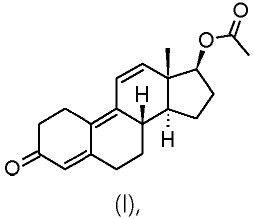
having a median value (D50) of the particle size distribution comprised in the range from 50 µm to 80 µm, or from 60 µm to 90 µm, or from 90 µm to 250 µm, by means of addition of a seed having a median value (D50) of the particle size distribution comprised respectively in the range from 8 µm to 15 µm, or from 15 µm to 20 µm, or from 20 µm to 90 µm, to a solution of Trenbolone Acetate in a solvent,
wherein said process comprises the following steps:
a) providing a solution of Trenbolone Acetate in a solvent or solvents mixture;
b) seeding said solution with a Trenbolone Acetate seed having a median value (D50) of the particle size distribution comprised in the range from 8 µm to 15 µm, or from 15 µm to 20 µm, or from 20 µm to 90 µm;
c) cooling until the obtainment of a suspension;
d) filtering the obtained suspension to obtain Trenbolone Acetate of formula (I) having a median value (D50) of the particle size distribution comprised in the range respectively from 50 µm to 80 µm, or from 60 µm to 90 µm, or from 90 µm to 250 µm,
wherein the solution of the step a) is Trenbolone Acetate in isopropanol.

2. Process according to claim 1, wherein the temperature during the cooling of the step c) decreases from a temperature comprised in the range from 20°C to 40°C to a temperature comprised in the range from of -20°C to -10°C.

3. Process according to any one of the claims from 1 to 2, comprising the previous step of preparation of the compound of formula (I): 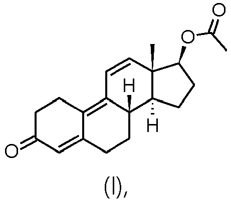 by acetylation of Trenbolone of formula (II):

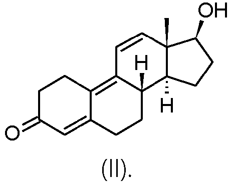

4. Process according to the claim 3, wherein the steps of the claim 1 are carried out one-pot.

## Patentansprüche

1. Verfahren zur Herstellung von Trenbolonacetat der Formel (I): 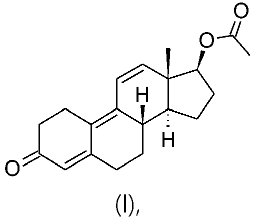
mit einem Medianwert (D50) der Partikelgrößenverteilung im Bereich von 50 µm bis 80 µm oder von 60 µm bis 90 µm oder von 90 µm bis 250 µm durch Zugabe eines Impfkristalls mit einem Medianwert (D50) der Partikelgrößenverteilung im Bereich von 8 µm bis 15 µm oder von 15 µm bis 20 µm oder von 20 µm bis 90 µm zu einer Lösung von Trenbolonacetat in einem Lösungsmittel,
das Verfahren umfasst die folgenden Schritte:
a) Bereitstellen einer Lösung von Trenbolonacetat in einem Lösungsmittel oder Lösungsmittelgemisch;
b) Beimpfen der Lösung mit einem Trenbolonacetat-Impfkorn mit einem Medianwert (D50) der Partikelgrößenverteilung im Bereich von 8 µm bis 15 µm, von 15 µm bis 20 µm oder von 20 µm bis 90 µm;
c) Abkühlen bis zur Bildung einer Suspension;
d) Filtern der erhaltenen Suspension, um Trenbolonacetat der Formel (I) mit einem Medianwert (D50) der Partikelgrößenverteilung im Bereich von 50 µm bis 80 µm, von 60 µm bis 90 µm oder von 90 µm bis 250 µm zu erhalten,
wobei die Lösung aus Schritt a) Trenbolonacetat in Isopropanol ist.

2. Verfahren nach Anspruch 1, wobei die Temperatur während des Abkühlens in Schritt c) von einer Temperatur im Bereich von 20°C bis 40°C auf eine Temperatur im Bereich von -20°C bis -10°C abnimmt.

3. Verfahren nach einem der Ansprüche 1 bis 2, umfassend den vorherigen Schritt der Herstellung der Verbindung der Formel (I): 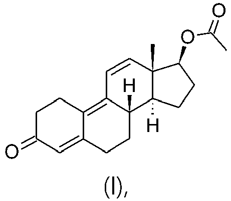 durch Acetylierung von Trenbolon der Formel (II):

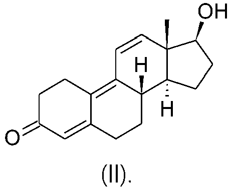

4. Verfahren nach Anspruch 3, wobei die Schritte des Anspruchs 1 in einem Eintopfverfahren durchgeführt werden.

## Revendications

1. Procédé de préparation de l'acétate de trenbolone de formule (I): 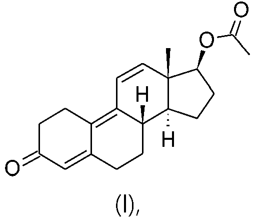
dont la granulométrie médiane (D50) est comprise entre 50 et 80 µm, ou entre 60 et 90 µm, ou entre 90 et 250 µm, par addition d'un germe dont la granulométrie médiane (D50) est comprise entre 8 et 15 µm, ou entre 15 et 20 µm, ou entre 20 et 90 µm, à une solution d'acétate de trenbolone dans un solvant,
le procédé comprend les étapes suivantes :
a) préparation d'une solution d'acétate de trenbolone dans un solvant ou un mélange de solvants ;
b) ensemencement de ladite solution avec un germe d'acétate de trenbolone dont la valeur médiane (D50) de la distribution granulométrique est comprise entre 8 µm et 15 µm, entre 15 µm et 20 µm, ou entre 20 µm et 90 µm ;
c) refroidissement jusqu'à obtention d'une suspension ;
d) filtration de la suspension obtenue pour obtenir de l'acétate de trenbolone de formule (I) dont la valeur médiane (D50) de la distribution granulométrique est comprise respectivement entre 50 µm et 80 µm, ou entre 60 µm et 90 µm, ou entre 90 µm et 250 µm,
la solution de l'étape a) étant de l'acétate de trenbolone dans de l'isopropanol.

2. Procédé selon la revendication 1, dans lequel la température lors du refroidissement de l'étape c) diminue d'une température comprise dans la plage de 20°C à 40°C à une température comprise dans la plage de -20°C à -10°C.

3. Procédé selon l'une quelconque des revendications 1 à 2, comprenant l'étape précédente de préparation du composé de formule (I) : 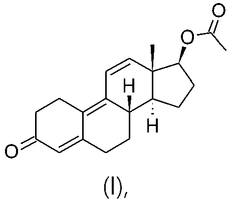 par acétylation de la Trenbolone de formule (II) :

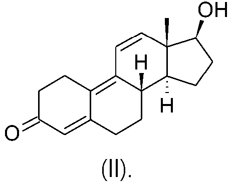

4. Procédé selon la revendication 3, dans lequel les étapes de la revendication 1 sont réalisées en un seul récipient.
